# DEMANDE DE BREVET EUROPEEN

(11) **EP 0 917 859 A1**
(43) Date de publication de la demande: **26.05.1999**
(21) Numéro de dépôt: 97870183.7
(22) Date de dépôt: 14.11.1997
(51) Int. Cl.: A61B 19/00

(54) **Dispositif de manipulation d'instruments chirurgicaux**

(71) Demandeur: Medsys S.A., 5030 Gembloux (BE)
(72) Inventeur: Andre, Jacques, 1410 Waterloo (BE); Mangez, Jean H., 5020 Namur (BE); Moreels, Xavier, 5030 Gembloux (BE); Polet, Roland, 1150 Bruxelles (BE)
(74) Mandataire: Van Malderen, Joelle

(57) **Abrégé**

Dispositif de manipulation d'un instrument chirurgical, caractérisé en ce qu'il comporte un support (1) comprenant au moins :
- un premier élément (4) articulé autour d'un premier axe horizontal (H1) parallèle au plan dans lequel est disposé le patient (PP');
- un second élément (5) articulé à l'extrémité du premier élément (4) autour d'un deuxième axe horizontal (H2) également parallèle au plan dans lequel est disposé le patient (PP');
- un élément déformable (6) fixé par un côté à l'extrémité du deuxième élément (5) et susceptible d'effectuer un mouvement de rotation autour d'un axe (C);
- un élément de préhension (7) fixé du côté opposé à celui du deuxième élément à l'élément déformable (6);
- un dispositif d'accrochage (8) d'un instrument chirurgical (9) à l'élément de préhension (7); et des moyens d'action (10 et 11) permettant d'effectuer des mouvements de rotation autour des différents axes (H1, H2 et C).

## Description

### Objet de l'invention

La présente invention se rapporte à un dispositif qui permet la manipulation, c'est-à-dire le positionnement et le déplacement, d'instruments chirurgicaux, tels qu'un endoscope, lors d'une opération chirurgicale.

### Etat de la technique

Il existe depuis de nombreuses années des aides se présentant sous la forme de dispositifs mécaniques pouvant porter des instruments chirurgicaux destinés à aider le chirurgien au cours d'opérations.

Des améliorations ont été apportées à ces aides, de telle sorte que ces dispositifs peuvent se déplacer et éventuellement actionner ces instruments chirurgicaux, et en particulier les scopes.

Ces dispositifs sont essentiellement destinés à libérer les mains du chirurgien afin qu'il n'ait plus à interrompre un acte pour déplacer par exemple le scope ou à devoir confier la manipulation de celui-ci à un assistant.

Néanmoins, la plupart des différents dispositifs connus n'offrent pas une précision dans le positionnement de l'instrument et une stabilité de maintien et/ou de déplacement suffisantes pour les applications dans le domaine médical telles que les actes chirurgicaux.

En particulier, on connaît un dispositif de stabilisation de l'endoscope tel que publié dans le document US-5184601, qui comprend une console indépendante munie d'un robot à axes multiples qui permet la réalisation de mouvements de rotation autour d'axes verticaux afin de couvrir un plan horizontal (XY) parallèle au patient. Ce dispositif présente les inconvénients de nécessiter une combinaison de plusieurs mouvements pour déplacer un scope selon les directions et orientations souhaitées par le chirurgien, et d'encombrer le champ d'action du chirurgien par la présence de bras se déplaçant dans le plan horizontal (XY) au-dessus du patient.

On connaît également par le document WO94/26167 un dispositif de positionnement destiné à porter un instrument pour des applications de chirurgie laparoscopique, qui prévoit un centre de rotation sphérique dudit instrument, ce centre étant constitué par le point de pénétration dans le ventre du patient, par exemple. Néanmoins, on observe que ce dispositif ne permettra pas la réalisation d'une translation selon l'axe de l'instrument, mouvement qui doit être rendu possible pour l'utilisation d'un scope.

Le document WO95/16396 décrit un dispositif de manipulation d'un instrument chirurgical à l'aide de plusieurs bras successifs qui effectuent des rotations autour d'axes verticaux ainsi que proposé ci-dessus, et qui, par un calcul complexe et détaillé des différents mouvements des actionneurs, permettra d'obtenir un déplacement incrémental de la tête du scope. Cependant, ces mouvements sont obtenus par trois changements de coordonnées cartésiennes successifs, ce qui rend la visualisation des mouvements relativement complexe. Afin de prévoir une aide décisionnelle à la manipulation de l'instrument, une commande à la voix peut également être proposée. Cette commande à la voix ne permet néanmoins pas la réalisation de petits déplacements rapides et/ou à cadence élevée.

### Buts de l'invention

La présente invention vise à proposer un dispositif de manipulation d'instruments chirurgicaux qui soit d'une conception relativement simple et d'un encombrement le plus réduit possible.

Un autre but de la présente invention vise à proposer un dispositif qui permette de libérer autant que possible les mains du chirurgien.

Un autre but de la présente invention vise à réduire autant que possible l'encombrement du champ d'action du chirurgien.

Un autre but de la présente invention vise à proposer un dispositif qui permette une combinaison de mouvements simples reproduisant les trois mouvements fondamentaux d'exploration du champ visuel.

Un autre but de la présente invention vise à proposer un dispositif de commande qui est simple et très peu encombrant, de telle sorte qu'il permette la manipulation d'autres instruments.

D'autres buts et avantages apparaîtront à la lecture du mémoire descriptif qui suit.

### Principaux éléments caractéristiques de la présente invention

La présente invention se rapporte à un dispositif de manipulation d'un instrument chirurgical composé d'un élément support, comprenant lui-même au moins:
- un premier élément articulé autour d'un premier axe horizontal parallèle au plan dans lequel est disposé le patient;
- un second élément articulé à l'extrémité du premier élément autour d'un deuxième axe horizontal également parallèle au plan dans lequel est disposé le patient;
- un élément déformable fixé par un côté à l'extrémité du deuxième élément et susceptible d'effectuer un mouvement de rotation autour d'un axe;
- un élément de préhension fixé du côté opposé à celui du deuxième élément à l'élément déformable;
- un dispositif d'accrochage d'un instrument chirurgical à l'élément de préhension; et
- des moyens d'action permettant d'effectuer des mouvements de rotation autour des différents axes.

De préférence, les premier et deuxième éléments se présentent sous la forme de bras articulés tandis que l'élément déformable se présente sous la forme d'un quadrilatère déformable et articulé.

Le deuxième élément articulé présente deux points d'articulation dont l'un peut coulisser dans une glissière et dont l'autre peut être bloqué ou libre.

Selon une forme d'exécution particulière, l'élément de préhension est disposé dans une glissière solidaire de l'élément déformable.

Selon une première forme d'exécution, l'élément support est fixé à la table d'opération à l'aide d'un dispositif de fixation coulissant sur un rail de fixation et qui comprend une ceinture, de préférence métallique, et ajustable en hauteur, serrée contre le support, ladite ceinture portant une clame supérieure fixe et une clame inférieure mobile qui coulisse dans une glissière, cette clame inférieure étant actionnée par un système bielle/manivelle permettant un serrage rapide des clames et permettant ainsi la fixation du dispositif de manipulation sur le rail de la table d'opération.

Selon une autre forme d'exécution préférée, ce dispositif de fixation comprend une vis, de préférence à filet, trapézoïdal, disposée verticalement au voisinage du centre de gravité de l'ensemble au moyen de deux supports très rigides et solidaires de l'élément support du dispositif de manipulation, ainsi qu'une poignée solidaire de la vis permettant d'actionner cette dernière en rotation par rapport à l'élément support, la rotation de la vis entraînant un déplacement vertical simultané des deux écrous, les écrous portant respectivement deux bras supérieur et inférieur à l'extrémité desquels sont soudées des mâchoires qui assurent une immobilisation rigide autour du rail de fixation de la table d'opération. Ce dispositif de fixation sera aisément inversé par rotation autour d'un axe vertical et ajusté par glissement le long du rail de fixation solidaire de la table d'opération afin de permettre le positionnement exact du manipulateur.

Le moyen d'accrochage de l'instrument chirurgical à l'élément de préhension est constitué par un joint passif comprenant lui-même au moins deux pièces articulées selon deux axes perpendiculaires d'une part à l'axe de l'instrument chirurgical et d'autre part à l'axe du premier élément, et dans lequel l'instrument chirurgical est fixé dans une position telle que le repère de verticalité de l'image dans le plan vertical soit assuré.

En outre, selon une forme d'exécution préférée, le manipulateur comprend un dispositif de commande constitué d'un mini-clavier à au moins six touches pour deux ou trois doigts, et dont la partie intérieure est de forme triangulaire. Ce dispositif de commande est complètement autonome et comporte un émetteur qui transmet les commandes au boîtier principal, cet émetteur se présentant sous la forme d'un petit boîtier de forme triangulaire.

### Description des figures

- La figure 1: représente une vue en perspective de la table d'opération sur laquelle le patient sera couché et à laquelle est fixé le dispositif de manipulation selon la présente invention.
- Les figures 2 et 3: représentent une vue en coupe latérale du dispositif de manipulation d'instruments chirurgicaux selon la présente invention pour deux applications d'interventions chirurgicales .
- Les figures 4 et 5: représentent des vues en coupe frontale du dispositif tel que représenté aux figures 2 et 3.
- La figure 6: représente une vue en coupe latérale correspondant à la figure 2 mais permettant de visualiser les différents mouvements du manipulateur.
- La figure 7: représente une première forme d'exécution du dispositif de fixation du manipulateur au lit du patient.

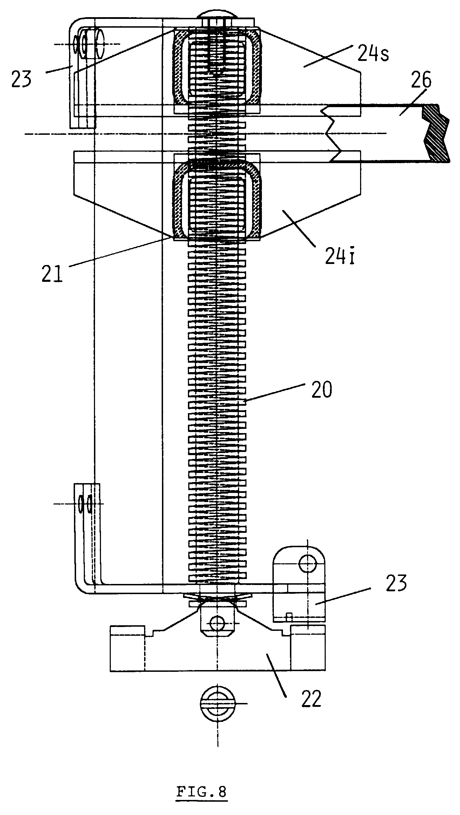

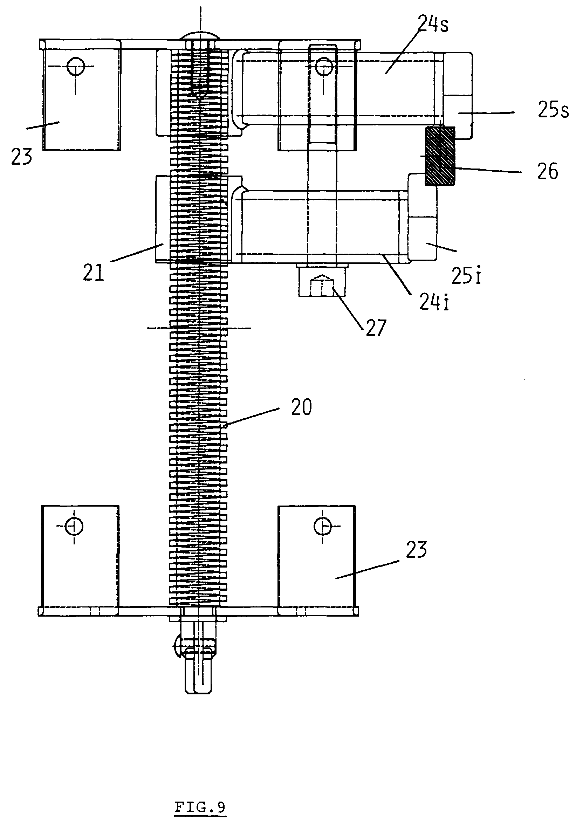

- Les figures 8 et 9: représentent une vue en coupe latérale et une vue en coupe par le dessus d'un dispositif de fixation permettant de fixer le manipulateur au lit du patient selon une seconde forme d'exécution.

### Description de plusieurs formes d'exécution préférées de l'invention

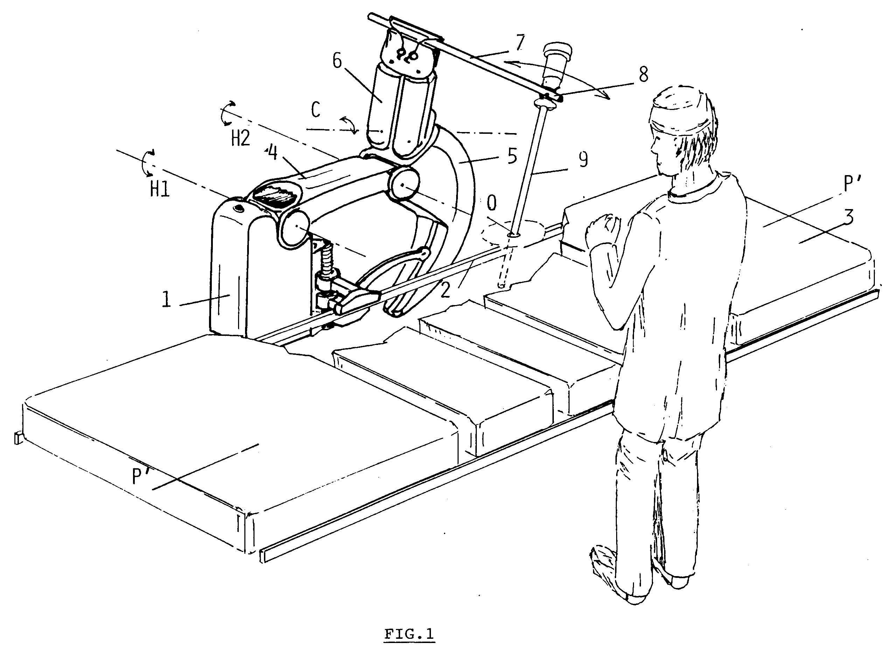

Le dispositif de manipulation selon la présente invention et représenté à la figure 1 en perspective est solidaire du lit (3) sur lequel le patient va se coucher.

Ce manipulateur présente l'avantage particulier qu'il possède un champ de déplacement tel que l'on peut l'appliquer à deux grandes familles d'interventions chirurgicales : d'une part les interventions chirurgicales gynécologiques et d'autre part les interventions chirurgicales abdominales. Il suffit, pour passer d'un champ à l'autre, de retourner simplement l'appareil par rapport à un plan vertical qui est perpendiculaire à l'axe selon lequel est couché le patient. Ceci signifie que l'appareil est bien sûr réversible et qu'il suffit de l'orienter selon la position avant ou arrière par rapport au patient.

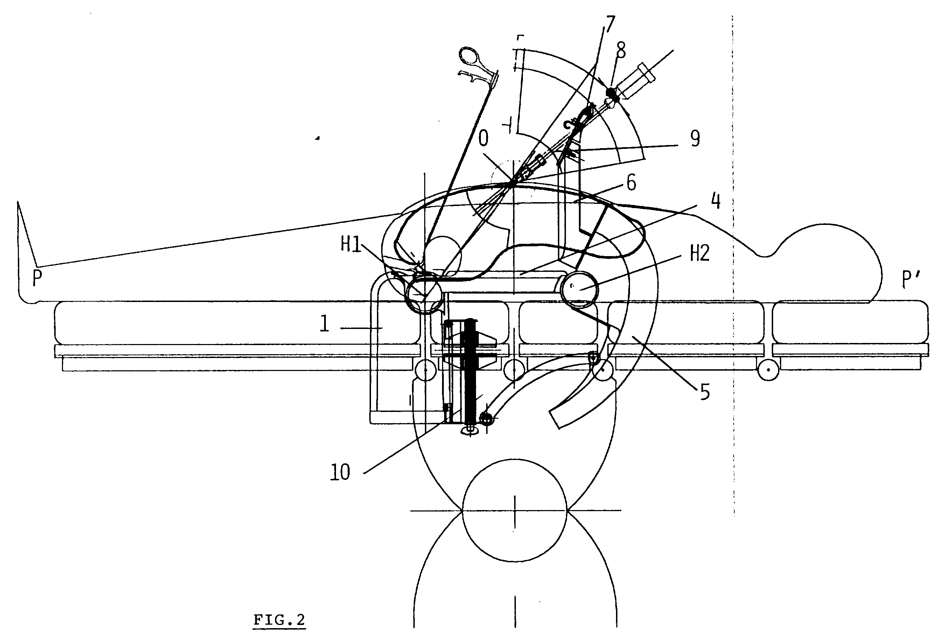

La figure 2 représente plus précisément le dispositif disposé de manière à permettre une intervention de type gynécologique. Ceci signifie essentiellement que l'endoscope pénétrera dans le patient dans une position telle que celui-ci permet d'obtenir une visualisation du champ où le chirurgien devra intervenir.

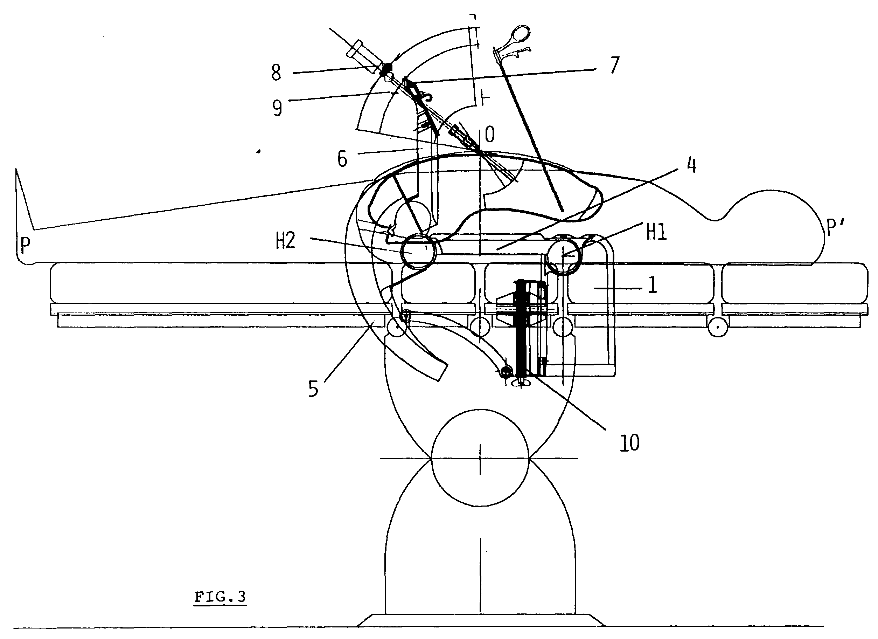

La figure 3 représente le dispositif disposé de manière à permettre une intervention de type abdominal. Dans ce cas, l'endoscope pénétrera dans le patient dans une position telle que celle-ci permet une visualisation vers la tête du patient.

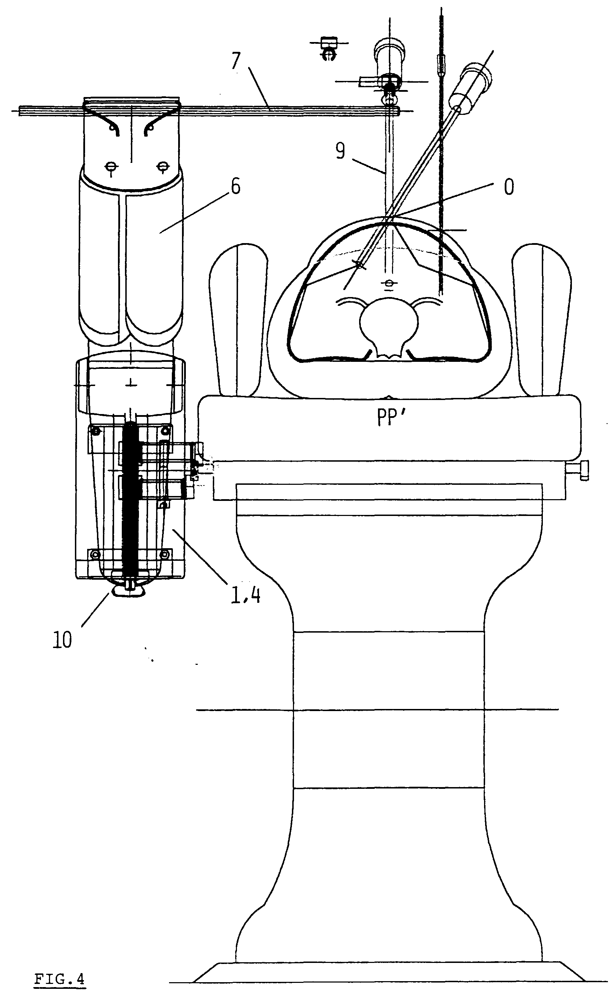

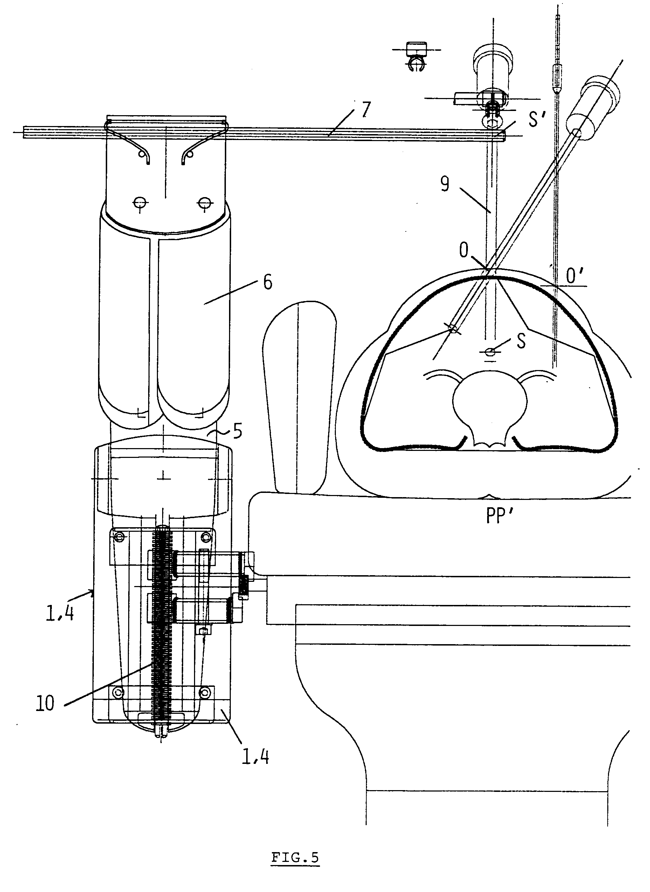

Dans les figures 1 à 6, le corps du patient est disposé selon un axe PP' avec la tête du patient dirigée vers P'. O représente le point de pénétration de l'instrument dans le ventre du patient. Ce point représente en général le nombril du patient.

Ainsi qu'il apparaît dans les diverses figures, ce manipulateur comprend au moins les éléments suivants :
- un support (1) ajustable en hauteur et destiné à être fixé sur un rail (2) solidaire de la table d'opération (3), ce support ajustable comprenant lui-même au moins :
   - un premier bras (4) articulé autour d'un axe horizontal H1 perpendiculaire au plan vertical passant par l'axe longitudinal du patient POP';
   - un deuxième bras (5) articulé à l'extrémité du premier autour d'un deuxième axe horizontal H2, également perpendiculaire au même plan POP';
   - un élément déformable (6) fixé par un côté à l'extrémité du deuxième bras (5) et susceptible d'effectuer un mouvement de rotation autour d'un axe C;
   - un bras de préhension (7) horizontal fixé au côté opposé du quadrilatère (6);
   - un dispositif d'accrochage (8) d'un instrument (9) au bras de préhension (7);
   - des actionneurs qui permettent d'exécuter des mouvements de rotation autour des différents axes mentionnés H1, H2 et C;
   - des moyens de pilotage et de synchronisation de ces mouvements; et
- une commande placée dans le creux de la main du praticien, lui permettant, au moyen de plusieurs touches, d'accéder aux différents mouvements.

Le manipulateur permet donc de déplacer un instrument (9) par des mouvements autour de deux axes de rotation horizontaux H1 et H2 perpendiculaires à un plan vertical POP' et autour d'un axe de rotation C.

La combinaison des mouvements permet d'obtenir les trois mouvements fondamentaux d'exploration du champ visuel, tels que représentés à la table 1.

**Table 1**

| **Mouvements de l'oeil** | **Mouvement donné au scope** | | |
|---|---|---|---|
| | **Direction** | **Sens** | **Symbole (+ sens)** |
| Accommodation (proche/lointain) | Zoom | in/out | ζ (i/o) |
| Rotation verticale (haut / bas) | Rotation | haut/bas | ρ (h/b) |
| Rotation horizontale (gauche / droite) | Latéral | gauche/droite | λ (g/d) |

Les mouvements produits correspondront donc à :
- ζ (i/o) :: déplacement suivant l'axe du scope (SS')
- ρ (h/b) :: rotation du scope dans le plan POP' autour de O
- λ (g/d) :: rotation du scope dans le plan S'OO' autour de O

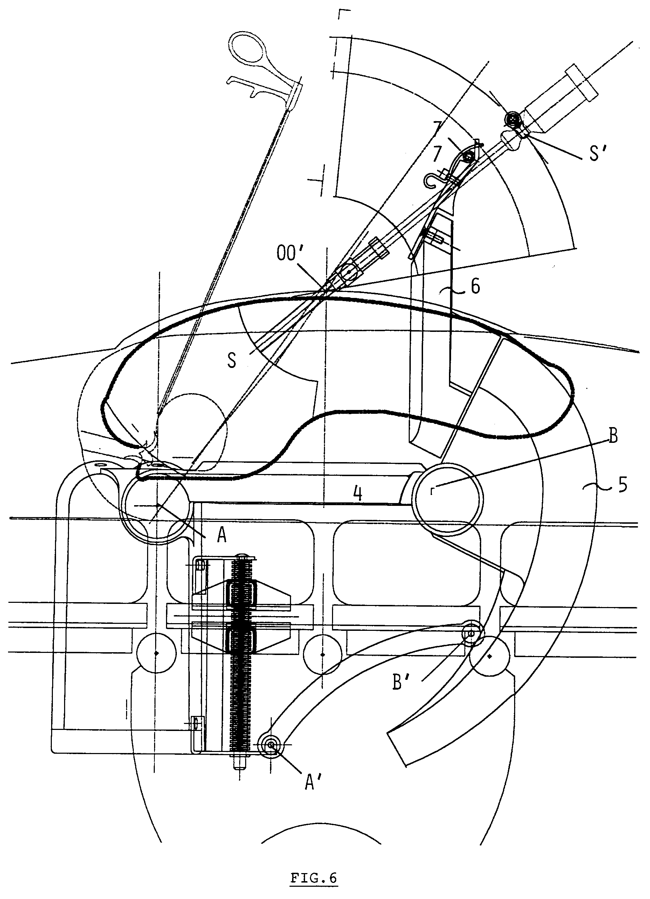

De préférence, le deuxième bras (5) se présente lui-même sous la forme d'un des côtés (BB') d'un parallélogramme articulé (ABB'A') tel que représenté plus en détail à la figure 6. L'articulation (B') peut coulisser suivant une glissière (51) solidaire du bras (5); la position par rapport au bras (5) et la longueur du bras (BB') peuvent donc varier.

Deux cas de figure peuvent se présenter :
- l'articulation (B) est bloquée : les bras et éléments (5, 6 et 7) sont donc simplement entraînés par la rotation du support (1) autour de l'axe H1, et le mouvement de S' et S est donc voisin de ρ;
- l'articulation (B) est libre, mais la position de (B') dans sa glissière est bloquée par un solénoïde (52) : dans ce cas, le mouvement du parallélogramme (6) entraîné par la rotation du support (1) autour de l'axe H1 induit un déplacement du bras de préhension (7), et donc de S' et S, voisin de ζ.

Cette exécution permet d'assurer les mouvements ρ et ζ au moyen d'un seul servomoteur.

Le bras de préhension (7) est toujours horizontal. Ceci permet de limiter au maximum l'encombrement créé par celui-ci dans le champ d'action du chirurgien. Il est relié au mécanisme et au lit du patient par le côté opposé au chirurgien.

L'instrument de chirurgie (9), tel qu'un endoscope, est relié au bras de préhension (7) par l'intermédiaire d'un joint passif (non motorisé) de manière à reproduire l'image que ce dernier perçoit et qu'elle soit toujours orientée correctement par rapport aux axes de référence du local, c'est-à-dire que les directions verticales doivent apparaître comme verticales sur le moniteur. La rotation du second bras (5) et la déformation du parallélogramme articulé (6) sont assurées par deux servomoteurs incorporés au bras (5).

La fixation du scope (9) au bras de préhension (7) en S' peut être assurée au moyen d'un joint passif constitué au moins de deux pièces articulées suivant l'axe (j) perpendiculaire à l'axe (d) du bras de préhension (7) et à SS'.

La pièce tenant le scope est fixée à ce dernier dans une position telle que le repère de verticalité de l'image soit dans le plan vertical passant par (j). De cette façon, l'image du scope apparaîtra toujours verticale pour un observateur fictif situé à la tête du patient et regardant parallèlement à l'axe PP'.

La pièce accrochée au bras de préhension (7) en S' est clipsée sur ce bras de préhension (7) de façon à pouvoir tourner librement autour de l'axe (d). Cette pièce est conçue pour pouvoir être déclipsée du bras de préhension (7) par simple action sur un petit levier.

En variante, cet ensemble peut être valablement réalisé en une seule pièce d'une matière élastique.

Le dispositif de commande du manipulateur peut être constitué d'un mini-clavier à six touches pour deux ou trois doigts (auriculaire et annulaire, et majeur pour la version à trois doigts) placé dans le creux de la main du praticien au moyen d'une pince adéquate entourant la main du praticien, caractérisée en ce que la partie intérieure (dans le creux de la main), de forme triangulaire, s'inscrive dans le "trapèze" de la main, entre l'éminence thénar et le pli des quatre phalanges, ne gênant ainsi aucunement les mouvements de la main.

De préférence, le bras de préhension (7) de l'instrument (9) peut être ajusté dans une glissière (71) de façon à disposer S' et donc S de manière qu'ils soient situés dans le plan POP'. Ceci constitue l'ajustement du point neutre de S en début d'opération. Grâce à cette particularité, on peut adapter le manipulateur à l'un ou l'autre type d'opération : abdominale ou gynécologique (voir figures 1 et 2) simplement en le tournant de 180° de dispositif de fixation et en inversant puis ajustant le bras de préhension (7) dans sa glissière (71).

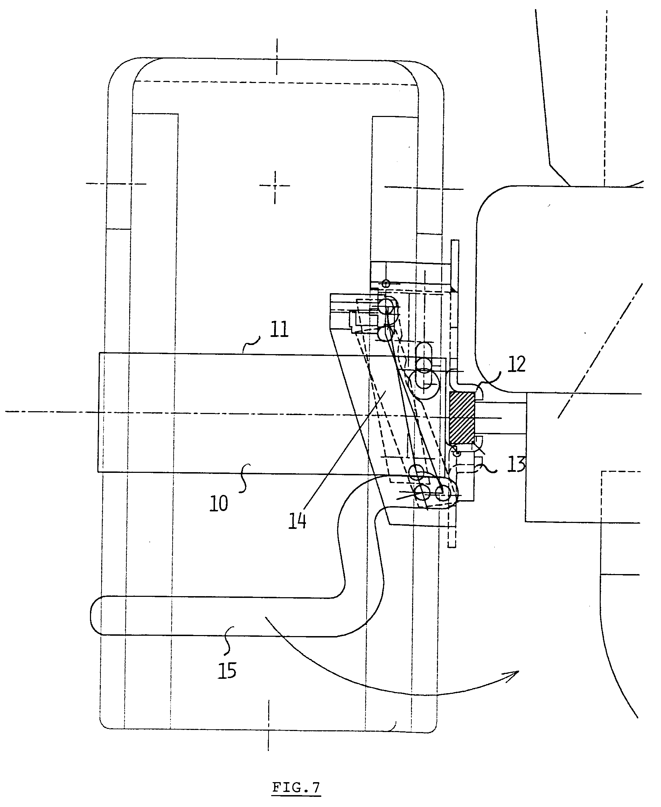

Selon une première forme d'exécution, un dispositif de fixation (10) du manipulateur à la table d'opération est représenté à la figure 7. Ce dispositif est constitué d'une ceinture métallique (11) pouvant être ajustée en hauteur et serrée contre le corps cylindrique du boîtier principal (1). Cette ceinture, lorsqu'elle est libérée, peut également être tournée de 180° par rapport au boîtier principal.

La ceinture porte une clame supérieure (12) fixe et une glissière (131) dans laquelle peut coulisser la clame inférieure mobile (13). Cette dernière est actionnée par un système bielle (14) / manivelle (15) permettant un serrage rapide des clames autour de la barre latérale de la table d'opération.

Le dispositif de serrage fonctionne par action "grip" (over-center latching).

Le dispositif de fixation (10) peut également permettre un ajustement à la fois par rotation autour d'un axe vertical et par glissement le long du rail de fixation (26) (voir figures 2 et 3).

Selon une autre forme d'exécution, le dispositif de fixation comprend une vis (20) à filet trapézoïdal, par exemple, qui est disposée verticalement au voisinage du centre de gravité de l'ensemble au moyen de deux supports (23) très rigides, solidaires du boîtier principal.

Une poignée (22) solidaire de la vis (20) permet d'actionner cette dernière en rotation par rapport à ses supports (23). La rotation de la vis (20) entraîne un déplacement vertical simultané des deux écrous (21). Ces écrous portent respectivement deux bras (24) supérieur et inférieur, à l'extrémité desquels sont soudées les mâchoires (25s et 25i) qui assurent une immobilisation rigide autour du rail (26). Ces mâchoires enserrent uniquement deux arêtes opposées du rail, permettant ainsi le dégagement aisé de l'ensemble par une légère rotation (20 à 25°) autour de l'axe du rail, vers la table.

L'immobilisation en rotation et en glissement est réalisée au moyen de la seule vis (27), qui par les deux bras (24), resserre à la fois les mâchoires (25) et les écrous (21).

L'inversion du manipulateur (chirurgie abdominale / gynécologique) se fait lorsque l'appareil est dégagé de la table d'opération, en tournant de 180° l'ensemble de clamage (21, 24, 25, 27) autour de la vis (20).

Le dispositif de commande peut en outre être parfaitement autonome. Il comporte un émetteur (par radio, infrarouge ou ultrason) qui transmet les commandes au boîtier principal. Cet émetteur tient dans un petit boîtier de forme triangulaire, comme décrit ci-dessus, et dont la hauteur (par exemple environ 20 mm) ne gêne pas le mouvement des doigts en cours d'opération. L'ensemble de commande peut être par exemple complètement immergé dans une résine pour le rendre étanche, permettant ainsi sa stérilisation dans un liquide adéquat. Une telle commande serait donc "semi-disposable", ayant une autonomie de vingt opérations par exemple.

## Revendications

1. Dispositif de manipulation d'un instrument chirurgical, caractérisé en ce qu'il comporte un support (1) comprenant au moins :
- un premier élément (4) articulé autour d'un premier axe horizontal (H1) parallèle au plan dans lequel est disposé le patient (PP');
- un second élément (5) articulé à l'extrémité du premier élément (4) autour d'un deuxième axe horizontal (H2) également parallèle au plan dans lequel est disposé le patient (PP');
- un élément déformable (6) fixé par un côté à l'extrémité du deuxième élément (5) et susceptible d'effectuer un mouvement de rotation autour d'un axe (C);
- un élément de préhension (7) fixé du côté opposé à celui du deuxième élément à l'élément déformable (6);
- un dispositif d'accrochage (8) d'un instrument chirurgical (9) à l'élément de préhension (7); et
- des moyens d'action (10 et 11) permettant d'effectuer des mouvements de rotation autour des différents axes (H1, H2 et C).

2. Dispositif selon la revendication 1, caractérisé en ce que les premier (4) et deuxième (5) éléments se présentent sous forme de bras articulés, tandis que l'élément déformable (6) se présente sous la forme d'un quadrilatère déformable et articulé.

3. Dispositif selon la revendication 2, caractérisé en ce que le deuxième élément (5) présente un point d'articulation qui peut coulisser dans une glissière (51) solidaire de l'élément déformable (6) tandis que l'autre point d'articulation peut être soit bloqué, soit libre.

4. Dispositif selon l'une quelconque des revendications précédentes, caractérisé en ce que l'élément de préhension (7) est disposé dans une glissière (71) solidaire de l'élément déformable (6).

5. Dispositif selon l'une quelconque des revendications précédentes, caractérisé en ce que l'élément support (1) est fixé à un rail (2) solidaire de la table d'opération (3) à l'aide d'un dispositif de fixation (10).

6. Dispositif selon la revendication 5, caractérisé en ce que le dispositif de fixation (10) comprend une ceinture de préférence métallique (11), et de préférence ajustée en hauteur, et serrée contre le support (1), ladite ceinture (11) portant une clame supérieure (12) fixe et clame inférieure mobile (13) qui coulisse dans une glissière (131), cette clame inférieure (13) étant actionnée par un système bielle (14)/manivelle (15) permettant un serrage rapide des clames (12 et 13) et permettant ainsi la fixation du dispositif à la table d'opération.

7. Dispositif selon la revendication 5, caractérisé en ce que le dispositif de fixation (10) comprend une vis (20), de préférence à filet trapézoïdal, disposée verticalement au voisinage du centre de gravité de l'ensemble au moyen de deux supports (23) très rigides et solidaires du support (1), une poignée (22) solidaire de la vis (23) permettant d'actionner cette dernière en rotation par rapport au support (1), la rotation de la vis (20) entraînant un déplacement vertical simultané des deux écrous (21), les écrous portant respectivement deux bras (24) supérieur et inférieur à l'extrémité desquels sont soudées des mâchoires (25s et 25i) qui assurent une immobilisation rigide autour de la table d'opération (26).

8. Dispositif selon l'une quelconque des revendications 5 à 7, caractérisé en ce que le dispositif de fixation (10) est inversé par rotation autour d'un axe vertical et est ajusté par glissement le long du rail de fixation (26a) solidaire de la table d'opération (26).

9. Dispositif selon l'une quelconque des revendications précédentes, caractérisé en ce que le moyen d'accrochage (8) de l'instrument chirurgical (9) à l'élément de préhension (7) est constitué par un joint passif comprenant lui-même au moins deux pièces articulées selon deux axes perpendiculaires d'une part à l'axe de l'instrument chirurgical (9) et d'autre part à l'axe du premier élément (4), et dans lequel l'instrument chirurgical est fixé dans une position telle que le repère de verticalité de l'image dans le plan vertical soit assuré.

10. Dispositif selon l'une quelconque des revendications précédentes, caractérisé en ce qu'il comprend un dispositif de commande (50) constitué d'un mini-clavier (51) à au moins six touches (52) pour deux ou trois doigts, et dont la partie intérieure est de forme triangulaire.

11. Dispositif selon la revendication 10, caractérisé en ce que le dispositif de commande est complètement autonome et comporte un émetteur qui transmet les commandes au boîtier principal, cet émetteur se présentant sous la forme d'un petit boîtier de forme triangulaire.

12. Utilisation du dispositif de manipulation d'un instrument chirurgical pour des interventions de type gynécologiques ou abdominales.
